# EUROPEAN PATENT SPECIFICATION

(11) **EP 2 456 327 B1**
(45) Date of publication and mention of the grant of the patent: **13.06.2018**
(21) Application number: 10737552.9
(22) Date of filing: 23.07.2010
(51) Int. Cl.: A23L 2/00, A61K 33/00, A61K 8/25, C02F 1/00, C02F 1/44, C02F 1/46, A23L 2/52, A23L 2/74, A61Q 3/00, A61Q 5/00, A61Q 19/00, A61K 8/19, C02F 1/469, A23L 2/78, C02F 1/68, C02F 9/00, C02F 103/02

(54) **A PROCESS FOR PRODUCING WATER ENRICHED WITH NATURAL ORTHOSILICIC ACID, STILL WATER OBTAINED BY THE SAID PROCESS, COSMETIC AND THERAPEUTIC USE OF SAID WATER**
EIN VERFAHREN ZUR HERSTELLUNG MIT NATÜRLICHER ORTHOKIESELSÄURE ANGEREICHERTES WASSERS, DAS DARAUS ERHALTENE STILLE WASSER, KOSMETISCHE UND THERAPEUTISCHE VERWENDUNG DES STILLEN WASSERS
UN PROCÉDÉ DE PRODUCTION D'UNE EAU ENRICHIE EN ACIDE ORTHOSILICIQUE NATUREL, L'EAU AINSI OBTENUE ET SON UTILISATION COSMÉTIQUE ET THÉRAPEUTIQUE

(30) Priority: 23.07.2009 WO PCT/IB2009/054216
(43) Date of publication of application: 30.05.2012
(73) Proprietor: SOCIETE ANONYME DES EAUX MINERALES D'EVIAN, "S.A.E.M.E", 74500 Evian Les Bains (FR)
(72) Inventor: DI GIOIA, Lodovico, F-91300 Massy (FR); JACQUEMONT, Marlène, F-91140 Villebon Sur Yvette (FR)
(74) Representative: Regimbeau
(86) International application number: PCT/EP2010/060730
(87) International publication number: WO 2011/009950

(56) References cited:
- EP-A- 0 391 318
- EP-A- 1 092 728
- US-A1- 2008 076 734
- GLYCAN SHOP-GLYCAN GROUP: "Le Silicium Organique G5 Glycan" INTERNET CITATION, [Online] pages 1-2, XP002524736 09-04-09 Retrieved from the Internet: URL:http://www.le-silicium-organique.com/b outique/115-silicium-organique- g5-1500mg-1.html> [retrieved on 2009-04-09]
- BAREL A ET AL: "Effect of oral intake of choline-stabilized orthosilicic acid on skin, nails and hair in women with photodamaged skin" ARCHIVES OF DERMATOLOGICAL RESEARCH ; FOUNDED IN 1869 AS ARCHIV FÜR DERMATOLOGIE UND SYPHILIS, SPRINGER, BERLIN, DE, vol. 297, no. 4, 1 October 2005 (2005-10-01), pages 147-153, XP019341162 ISSN: 1432-069X
- DATABASE WPI Week 199237 Thomson Scientific, London, GB; AN 1992-304480 XP002560110 & JP 4 210288 A (JAPAN ORGANO CO LTD) 31 July 1992 (1992-07-31)
- DATABASE GNPD [Online] MINTEL September 2006 ANONYMUS: 'Natural Mineral Water' Database accession no. 588004
- Ravin Jugdaohsingh ET AL: "Dietary Silicon Intake Is Positively Associated With Bone Mineral Density in Men and Premenopausal Women of the Framingham Offspring Cohort", Journal of Bone and Mineral Research, vol. 19, no. 2, 16 February 2004 (2004-02-16), pages 297-307, XP055389888, US ISSN: 0884-0431, DOI: 10.1359/JBMR.0301225
- LOEPER J ET AL: "THE ANTIATHEROMATOUS ACTION OF SILICON", ATHEROSCLEROSIS, ELSEVIER, AMSTERDAM, NL, vol. 33, 1 January 1979 (1979-01-01), pages 397-408, XP000612679, ISSN: 0021-9150, DOI: 10.1016/0021-9150(79)90032-7
- GONZALEZ-MUNOZ ET AL: "Beer consumption reduces cerebral oxidation caused by aluminum toxicity by normalizing gene expression of tumor necrotic factor alpha and several antioxidant enzymes", FOOD AND CHEMICAL TOXICOLOGY, PERGAMON, GB, vol. 46, no. 3, 17 November 2007 (2007-11-17), pages 1111-1118, XP022441221, ISSN: 0278-6915
- D.M Reffitt ET AL: "Orthosilicic acid stimulates collagen type 1 synthesis and osteoblastic differentiation in human osteoblast-like cells in vitro", BONE, vol. 32, no. 2, 1 February 2003 (2003-02-01), pages 127-135, XP055389931, GB ISSN: 8756-3282, DOI: 10.1016/S8756-3282(02)00950-X

## Description

The invention relates to a method for obtaining still drinking water with an increased content of natural bioavailable orthosilicic acid and substantially unchanged content of valuable dissolved mineral ions. Said drinking water can be used to produce beverages.

In the last two decades, a lot of studies have proved the health benefits of drinking natural water. The major minerals dissolved in water are calcium, magnesium, sodium, potassium, bicarbonate, sulfate, chloride, nitrate, fluoride and silicon. Most of them have also some specific functions in human body such as calcium for bones and blood pressure.
More particularly, silicon is a mineral that was recently defined as essential in humans, by participating to the normal metabolism of superior animals (Carlisle EM. Silicon. In: O'Dell B.L., Sunde RA, eds. Handbook of nutritionally essential mineral elements. New York Basel: Marcel Dekker, Inc. 1997:603-618).
Through its action on collagen and elastic fibbers, silicon has been demonstrated to play a key role in the physiology of connective tissues, and as a consequence in the functions of various organs, *i.e*. mainly tegument, bones, and blood vessels (Carlisle EM. In vivo requirement for silicon in articular cartilage and connective tissue formation in the chick. Journal of Nutrition 106, 478-484, 1976 ; Carlisle EM. Biochemical and morphological changes associated with long bone abnormalities in silicon deficiency. Journal of Nutrition 110, 1046-1056, 1980 ; Calomme MR. & Berghe DA. Supplementation of calves with stabilized orthosilicic acid. Effect on the Si, Ca, Mg, and P concentrations in serum and the collagen concentration in skin and cartilage. Biological Trace Element Research 56, 153-165, 1997). It improves force and brightness of hairs; nails strength, quality, and protection against infections. On skin, silicon has been shown to increase strength, elasticity, and hydration; as well as to protect skin against diseases thanks to its anti-inflammatory properties; and finally to prevent wrinkles through its action on collagen synthesis. Some authors considered silicon as essential to the formation of bone matrix and bone mineralization (Carlisle EM. Silicon as an essential trace element in animal nutrition. Ciba Foundation symposium 121, 123-139, 1986). Many supplementation studies have shown an increased bone volume in women suffering from osteoporosis or having a bone weight deficit. An epidemiological study has shown a better ossification of the hip in men consuming more silicon and in premenopausal women (Jugdaohsingh, the journal of Nutrition, Health & Aging, vol 11, n°2, 2007, pages 99-110).
Several studies have revealed the anti-atherosclerotic activity of silicon in animals and humans (Schwarz K., Ricci BA., Punsar S. & Karvonen MJ. Inverse relation of silicon in drinking water and atherosclerosis in Finland. Lancet 1, 538-539, 1977 ; Schwarz K. Silicon, fibre, and atherosclerosis. Lancet 1, 454-457, 1977 ; Loeper J., Goy L., Rozensztajn L. & Fragny M. The antiatheromatous action of silicon. Atherosclerosis 33, 397-408, 1979). If the mechanism remains still shadowy, and needs to be studied further, authors suspect that this property is linked to preservation, by silicon, of endothelial matrix structure and function under atheromatous conditions.
Beside these effects, silicon is recognized as a treatment for gastric hyper-acidity. Silicon has also been considered as preventing aluminium toxicity by sequestering aluminium and therefore preventing cognitives impairment and neurodegenerative diseases such as Alzheimer's disease (Exley et al., Journal of Alzheimer's Disease, 10, 2006, pages 17-24, Gilette-Guyonnet et al., Am J Clin Nutr, 2005, 81, pages 897-902).
Silicon (Si) is very abundant on earth, making up 26% of the earth crust by weight and is present in almost all of the earth's minerals rocks, sands and clays. It exists as silicon dioxide, also called silica [SiO₂], in an amorphous polymerized state (colloidal silica) or crystalline (quartz), but also in the form of silicates SiO₄⁴⁻ associated in the rocks to the cations K⁺, Na⁺, Ca⁺⁺, Mg⁺⁺, Fe⁺⁺, Fe³⁺, Al³⁺. The dissolution of rocks containing silicon is very slow and produces orthosilicic acid (OSA), which chemical formula is [Si(OH)₄], having a solubility in water around 120 mg/l at 25°C and neutral pH. This solubility is conventionally expressed in milligrams of SiO₂ per liter of water, and this convention is adopted in the following description (the conversion factor is: 1 mg/l of SiO₂ = 1.6 mg/l of Si(OH)₄). OSA is a weak diacid (pKₐ₁ = 9.6 and pKₐ₂ = 12.6) which is under undissociated form at neutral or acid pH. The orthosilicic acid (OSA) has a good bioavailability. It is readily absorbed to > 50% of the total ingested amount (Martin, The Journal of Nutrition, Health & Aging, vol 11, N° 2, 2007, pages 94-98).
Although OSA is very bioavailable, its concentration depends on type of water. The richest waters are groundwaters from volcanic stones that contain 30 to more than 100 mg/l of OSA (Volvic 32 mg/l and dry residue: 134 mg/l, Fiji 85 mg/l and dry residue: 218 mg/1) and thermo-mineral waters which offer 50 to more than 100 mg/l due to the higher solubility of OSA at higher temperature. But, these natural drinking waters are rare. Moreover, groundwaters containing more than 50mg/l of OSA are even rarer and contain only low amount of other valuable soluble minerals. Another disadvantage is that thermo-mineral waters generally have on the contrary a too high mineral content. Their high mineral content and in particular sodium, makes them unadapted to a regular consumption as drinking water because kidneys and blood pressure could be affected. On the contrary, groundwaters from intrusive stones have lower concentrations of OSA (10 to 30 mg/1), similar to surface waters and groundwaters from shallow aquifers (<10 mg/1) or also sea water (1 to 3 mg/1).
Of special interest are natural waters with a low or medium mineral content (less than 1100 mg/l of dissolved minerals and in particular less than 20 mg/l of sodium) and with a high content of silicon.
Today, functional waters and beverages are getting increasing interest from the consumers because they can help to prevent long term health problems. When they have the choice, consumers always prefer the natural products and those with low/no calories, like water.

Different companies try to develop process for producing water enriched with silicon.

Some biological preparations comprising OSA and a polypeptide as complexing/stabilizing agent have already been proposed such as for example in EP1092728, US20060165815 or CN101279737. These biological preparations contain choline and other substances with an osmolyte effect, such as animal gelatin, betaine, glycine or taurine, and also some sugars. They are soluble and can be dissolved in drinking water. They have the drawback of not being natural due to the added organic molecules that can also bring calories along with OSA. One example of commercial product is BioSil™, containing 2% silicon, 47% choline chloride, 33% glycerine and 18% distilled water.
In JP200626619, the author proposed a mineral water with added metasilicic acid (H₂SiO₃). This water is produced by dissolving some special stones with a thermal treatment up to 90-100°C and waiting 1 hour minimum. The major drawbacks of this invention are the amount of energy required to let water boil such a long time and the difficulty of producing such water on an industrial scale.
Another possible way of adding silicon to water is by dissolving soluble silicate salts, but the pH of the fortified water is alkaline and will require an acid neutralisation.
A second possible solution is the addition of colloidal silicon gel in water in the form of large, insoluble, polymer molecules of silicic acid suspended in water. However, these polymer-molecules cannot be absorbed directly through the stomach wall and therefore have a low rate of absorption. Moreover, silicate salts and colloidal silicon gel are not natural ingredients.
A well know process for concentrating substances dissolved in water is reverse osmosis (RO), in which the feed water flow is separated into a permeate, almost pure water, and a retentate, containing almost all the initially dissolved substances. For example Koprivnjak et al. (Water Research 40:3385-3392, 2006) proposed RO as an effective method for concentration of natural organic matter from fresh river waters. A pre-treatment by using an H⁺-saturated cation exchange resin was applied to remove some ions that can precipitate during RO process, such as calcium and bicarbonate forming insoluble CaCO₃. The co-concentration of remaining inorganic substances and silica was considered by the authors as a negative effect of RO. For that reason, an electrodialysis (ED) process was associated to RO, in order to non-specifically remove ions from the retentate. In order to partially remove the silica, the pH was adjusted to 9.2 - 9.6, before ED, so that OSA is partially converted into its conjugate base H₃SiO₄⁻ An electrodialysis equipment comprises a cathode at one terminal end, an anode at the opposite end and in between alternating pairs cation selective (to be understood cation permeable) and anion selective (to be understood anion permeable) standard grade membranes forming a plurality of diluting and concentrating chambers. Clearly, and in particular because of the ED membranes used, the goal of achieving a natural drinking water with substantially unchanged valuable mineral content is not achieved. Vetter et al. (Separation and Purification Technology 56:383-387, 2007) used the same association to isolate dissolved organic matter from seawater, with no reference to silicon or single ions behaviour.
Another nice association of RO and ED is described in FR2818267, in which a process for depleting monovalent cations from water is described. The inventors observed that the selectivity of electrodialysis between the monovalent cations and the divalent cations may increase in proportion as the ionic concentration of the water to be treated increases. However the scope of the invention was not to increase silicon content and silicon is not mentioned. Furthermore the type of ED membranes used was not specifically selected. Therefore, it was not possible with the process described in this patent application to obtain a natural drinking water with a higher amount of silicon and with substantially unchanged valuable mineral content.
Another association of RO and ED is described in US20080277341, with the purpose of making RO permeate water and mineral water from deep seawater. The inventors obtain a mineral water with high mineral content (example: sodium ion 200-5000 mg/1). Silicon behaviour in the process and final content is not mentioned. Furthermore the type of ED membranes used was not specifically selected. Turek (Desalinisation 153:377-381, 2002) proposed a two-step electrodialysis of seawater, the first step being equipped with a stack containing only univalent ion permeable membranes and the second step with a stack containing only standard grade membranes , in order to avoid gypsum (CaSO₄). Therefore none of the stack contains at the same time univalent permeable membranes and standard grade membranes. Furthermore, this process does not describe the use of RO and silicon is not mentioned.

EP 0 391 318 describes a way of recovering some minerals from sea water, by adding chemicals such as acid and alkali agents, and thermal / filtration treatments to remove most of the water. These minerals must then be diluted in water for final use in food and beverage preparations. The inventor of this document neither describes initial composition of sea water, nor that of dilution water. The process of this document will modify some valuable mineral ions such as sulfate, bicarbonate, sodium and calcium due to addition of sulfuric acid (sulfate added + decomposition of bicarbonate into carbon dioxide) and sodium hydroxide (sodium added) or sodium hydroxide + calcium oxide (also calcium added).
Furthermore it should be pointed out that sea water has a very low level of silicon (1 to 2 mg SiO₂ / L). The ion water described in this document is obtained by dissolving precipitate (b) in water at about 100 g / L (stock solution). This stock solution is then diluted 20 times to reach pH 10. Water at pH 10 is considered non potable by WHO. The stock solution is diluted 100 times to reach a pH 8.3, acceptable for potable water (see example 2 in this document). Considering Si content of 697 mg/kg in (b) (see table 1), and assuming a density of (b) close to 1, ion water will contain around 70 mg Si / L and preparation of example 2, only 0.7 mg Si/L after dilution of 100 times. This is equal to 1.5 mg SiO₂ / L, i.e. exactly the initial content in sea water. Thus, process described in this document doesn't provide a silica enriched water. Furthermore the process described in this document has the following drawbacks:
It can only be used on sea water.
It is only a batch process requiring very long contact times (2-3 hours then 10 hours).

It requires addition of chemicals (dangerous chemicals such as strong acids and alkali are at risk of handling) and the pH of water is substantially modified.
It is complex and difficult to carry out on industrial scale, with the steps of heating water (energy expensive) and getting precipitates (difficult to manage for industrial plants, especially to clean).
EP 1 092 728 describes a complex containing orthosilicic acid which can be used in water in order to enrich water with orthosilicic acid. However the complex contains polypeptides which are therefore present in the enriched water obtained using this complex. Therefore the water does not contain only natural orthosilicic acid originating from the initial water but contains other components since the orthosilicic acid has an exogenous source (such as tetraalcoxysilane). Therefore the water obtained using this complex should also contains preservatives to keep it microbiologically safe during storage and should be stored cold since the organic molecules added could constitute a source of food for pathogenic bacteria. Therefore the complex described in EP 1 092 728, when added to water does, not provide a calories free, preservatives free, allergen free, pH neutral and totally natural hydration solution.

Therefore, it is a goal of the present invention to find a novel process for preparing a water or a beverage with increased content of natural soluble and bioavailable silicon in the form of orthosilicic acid, without substantially modifying the content of dissolved valuable mineral ions such as calcium, magnesium, sodium, potassium, bicarbonate, sulfate or chloride
One of the aspects of the invention is to propose a new process for preparing a water or a beverage that preserve the light taste and the hydration properties of the water, while bringing additional benefits of high OSA level.

The present invention relates to a process for producing water enriched with natural orthosilicic acid, said process comprising at least the following successive steps:
a. a step of reverse osmosis of a raw water containing a low amount of orthosilicic acid in order to obtain a retentate;
b. a step of electrodialysis of the retentate obtained in step a with an electrodialysis stack comprising alternating pairs of cation selective membranes and anion selective membranes, said cation selective membranes and anion selective membranes being selected in the group of monovalent selective membranes and standard grade membranes such that the stack contains at least one cation standard grade membrane, one cation monovalent selective membrane, one anion standard grade membrane and one anion monovalent selective membrane;
c. a step of recovery of a final water having a orthosilicic acid weight content equal to at least 150%, particularly at least 200%, more particularly at least 300% of the orthosilicic acid weight content of the raw water, and a dissolved valuable mineral ions weight content comprised in the range of 70 to 130%, particularly in the range of 80 to 120%, more particularly in the range of 90 to 110 % of the dissolved valuable mineral ions weight content of the raw water. The term "natural orthosilicic acid" as used herein, designates orthosilicic acid naturally presents in water, which chemical formula is [Si(OH)₄], and having a solubility in water around 120 mg/l at 25°C and at neutral pH.

The process according to the present invention has at least one of the following advantages:
It can be used on any type of water, including on water different from sea water;
It can be used in a continuous mode;
It doesn't require addition of chemicals (dangerous chemicals such as strong acids and alkali are at risk of handling), only natural minerals are recovered in the final water and the pH of water is not substantially modified;
The initial content of minerals and increased content of silicon can be recovered in the final water obtained by this process without adding chemicals and without adding dilution water;
The process is simple and easy to carry out on industrial scale.

The expression "dissolved valuable mineral ions" as used herein, designates the major minerals dissolved in water that have a specific function in human body, which are in particular calcium, magnesium, sodium, potassium, bicarbonate, sulfate, and chloride ions.
The expression "raw water containing a low amount of orthosilicic acid" as used herein, designates raw water containing less than the desired amount of orthosilicic acid, in particular less than 45 mg/L of SiO₂, more particularly less than 40mg/L of SiO₂, still more particularly less than 30 mg/L of SiO₂, further still more particularly less than 20 mg/L of SiO₂, for example less than 10mg/L of SiO₂.
The term "electrodialysis stack" above mentioned doesn't designate the cation selective membranes present between each electrode and the electrodialysis stack in order to isolate the electrolyte ; The addition of at least one cation selective membrane at each extremity is well known in the prior art.

In a particular embodiment, the process comprises a preliminary step α) before step a) consisting in the decontamination of the raw water in order to obtain a decontaminated raw water and in that step a) is carried out on the decontaminated raw water. The pre-treatment is preferably carried out by well known techniques that can remove the mineral or organic contaminants without modifying the content of valuable dissolved mineral ions, such as using activated carbon to remove organic contaminants or metal oxides and zeolites filtration media to remove mineral contaminants and unstable elements such as iron or manganese. However, no treatments by ion exchange resins and no injection of antiscaling agents or of any type of reagent to increase or decrease pH is carried out on raw water or on decontaminated raw water during step α) or before step a), in order to recover in the retentate of reverse osmosis all and only the original and natural minerals present in the raw water.

The term "decontaminated water" as used herein, designates a water which complies with the drinking water regulations and standards and in particular with a level of contaminants well below the drinking water standards and preferably with a non-detectable level of contaminants.

In another particular embodiment, the raw water is selected among groundwaters or surface water, more particularly spring water, mineral water, natural mineral water or drinking waters.
The term "natural water" as used herein, designates water that is extracted from protected hydrogeological underground systems and is free from contaminants of human origin.
The term "drinking water" as used herein, designates water conforms to national or European standards. For example, in Europe, the drinking water is defined in directive 98/83 EC. This directive specifies that chloride and sulfate should be below 250 mg/l each and sodium below 200 mg/L, in order to avoid impaired taste. High sulfate content in drinking water can result in gastrointestinal disorders. The term "sodium free" is used both in United States and Canada, and more recently approved in Europe, for waters containing less than 20 mg/l of sodium.

In an advantageous embodiment, the final water has an orthosilicic acid content comprised between 30 and 120 mg/L of SiO₂, particularly between 45 and 120 mg/L, in particular between 55 and 120 mg/L, for example between 65 and 120 mg/L, more particularly between 75 and 120 mg/L.
It should be understood that the final water obtained at the end of the process is a water enriched with natural orthosilicic acid, therefore having an orthosilicic acid weight content higher than the raw water.
In a particular embodiment, the final water has a dry residue below 1500 mg/l, particularly below 1100mg/L, more particularly below 500mg/L.

The term "dry residue" as used herein, designates the sum of the water weight content in calcium, magnesium, sodium, silicon, potassium, nitrates, sulfates and chloride ions, plus the half water weight content in bicarbonate ions.

In another particular embodiment, the final water has a stable orthosilicic acid weight content at storage temperature, even in particular at a temperature below 10°C, more particularly of about 4°C.

In another advantageous embodiment, the process comprises a final step d) after step c), consisting in the addition of the final water to a beverage and/or a syrup and/or a powder, in order to form a beverage enriched with natural bioavailable orthosilicic acid.
The final water can be mixed with different ingredients such as mineral or organic salts, sugar, natural or artificial sweeteners, flavours, acids, preservatives, colouring agents, vitamins, plant extracts, juices, proteins and/or fibers.

In another particular embodiment, the recovery rate of reverse osmosis is adjusted to keep saturation rate of insoluble minerals in the retentate below 100%, particularly below 90%, more particularly below 80%.
The reverse osmosis is controlled in order to have a recovery rate of retentate sufficiently low to keep the concentration of insoluble salts, such as CaCO₃, MgCO₃, Mg(OH)₂, CaSO₄, BaSO₄, SrSO₄, CaF₂ and SiO₂ below the limit of saturation thus avoiding a reverse osmosis membrane scaling without adding antiscaling agents or removing valuable dissolved mineral ions before the reverse osmosis step.

More advantageously, the reverse osmosis membranes are selected among hot sanitizable membranes, sea water desalination membranes, brackish water membranes, fouling resistant membranes and low energy membranes, in particular among brackish water membranes and sea water desalinisation membranes.

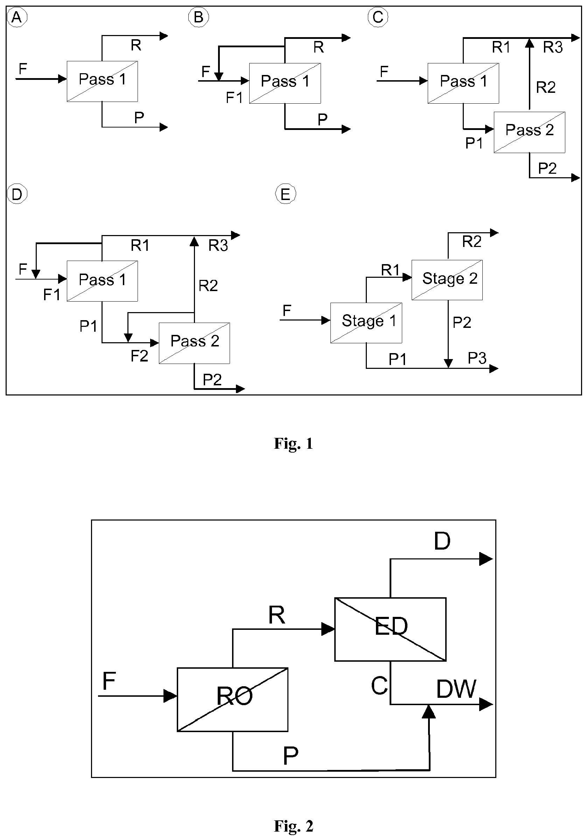

The term "hot sanitizable membrane" as used herein, designates membrane that can be sanitized and sterilized by using hot water at low pressure, thus avoiding the use of chemical sterilizing agents that could subsequently contaminate the retentate if not correctly flushed out of the system.
The term "low energy membrane" as used herein, designates membrane that can be operated at low pressure in order to save energy.
RO membranes can be made of different materials such as cellulose acetate or polyamide. Polyamide membranes are particularly advantageous as they have very good rejection of OSA (Lee Comb, RO plays a major role in silica removal, Water Technology Magazine, Volume 22, Issue 12, 1999). Commercial RO elements are made of spiral wound membranes and are designed for different working pressures : sea water elements works at 25-90 bars, brackish water elements at 10-30 bars and low energy elements at 7- 15 bars. For the purpose of the invention, any type of RO element is suitable, but elements working at pressure lower than 25 bars are more advantageous, in order to save energy cost. Low energy sanitary elements such as SanRO ESPA2 from Hydranautics or HSRO from Filmtec are of particular interest because they can efficiently concentrate minerals and OSA in the retentate at low operating pressure while avoiding bacteria contamination. Additional advantage of these elements is the possibility to sanitize them by using hot water, thus avoiding the use of chemicals that would subsequently contaminate the retentate. In one advantageous embodiment of the invention, the recovery rate of the permeate is kept below 75% in order to increase the rejection of minerals and OSA in the retentate above 90%. Without limitations, many configurations of RO can be used in the invention, such as 1 pass RO with or without recirculation loop of retentate (R) to feed (F) to form a feed F1 (Figure 1A and B) or 2 passes RO with or without recirculation loops of pass 1 and / or pass 2 retentate R1 / R2 to feed F / permeate PI to form feeds F1 / F2 (Figure 1C and D). Advantageously, a 2 passes system ensures the maximum recovery of dissolved minerals and OSA in retentate R3, by recovering dissolved minerals and OSA not removed by pass 1 and still present in permeate PI. More advantageously, energy savings can be made by adding one additional stage to pass 1 (Figure 1 E).
In another advantageous embodiment, the rejection rate of dissolved mineral ions and orthosilicic acid in the retentate is at least higher than 80%, particularly higher than 90 %, more particularly higher than 95 %.
The principle that governs electrodialysis is an electrical potential difference across an electrodialysis stack comprising alternating series of cation and anion exchange membranes between an anode and a cathode. As known by the one skilled in the art, at least one cation exchange membrane is present between each electrode and the electrodialysis stack. The feed solution containing both positive and negative ions enters the membrane stack to which a voltage is applied, thus causing the migration of the ions toward their respective electrodes. The cation exchange membranes (also called cation selective) allow the transfer of cations but inhibit the transfer of anions. Conversely, anion exchange membranes (also called anion selective) allow the transfer of anions but inhibit the transfer of cations. The result is alternating compartments containing streams of dilute ion concentration (diluate) and streams rich in ion concentration (concentrate). An ionic rinse solution is circulated past the electrodes to maintain conductivity of the membrane stack while preventing potentially corrosive ions from the feed solution from contacting the electrodes.
Metal electrodes are made of very resistant titanium plates covered by a layer of metal oxide, such as platinized titanium, but 316 stainless steel is also possible. In the process of the invention, it is not necessary that both electrodes (anode & cathode) are made of titanium. Advantageously, only the anode is made of platinized titanium. The cathode can simply be made of stainless steel, thus reducing significatively the cost of the process. More advantageously, all types of electrodes used in the process of the invention are food grade, which is the case of stainless steel and platinized titanium materials.
The desirable characteristics of membranes used in electrodialysis applications include selectivity between ions of opposite charge, high ionic conductivity, low electrical conduction, long-term chemical stability, mechanical strength, and resistance to fouling. These characteristics are determined by the membrane matrix polymer and the fixed ionic moiety that affect the ion selectivity of the membrane. Polymer materials such as polystyrene, polyethylene, and polysulfone are often chosen for the membrane matrix and are often cross-linked to ensure stability. Fixed ionic moieties are for example alkyl ammoniums for anion selective membranes, such as ∼NR₃⁺, ∼NHR₂⁺, ∼NH₂R⁺, and sulfonate ∼SO₃⁻ or carboxylate ∼CO₂⁻ for cation selective membranes. Non limitative examples of ion-exchange membranes suitable for the process of the invention are Selemion® produced by Asahi Glass Company (Japan) or Neosepta® produced by Astom Corporation (Japan). Neosepta® AMX and CMX membranes are standard grade, general-purpose polystyrene-based ion exchange membranes. Neosepta® CMXS membranes have a thin cationic charged layer on the membrane surface that increases the selectivity for monovalent cations compared to multivalent cations. As a consequence, when such type of cation monovalent selective membranes (CMS) are used, monovalent cations such as for example Na⁺ is more removed from diluate and divalent cations such as for example Mg²⁺ is less removed, compared to cation standard grade type of membranes (CSG). Neosepta® ACS membranes have an increased surface cross-linking that increases the selectivity for monovalent anions compared to divalent anions. As a consequence, when such type of anion monovalent selective membranes (AMS) are used, monovalent anions such as for example Cl⁻ is more removed from diluate and divalent anions such as for example SO₄²⁻ is less removed, compared to anion standard grade type of membranes (ASG). Advantageously, all the membranes used for the purpose of the invention are of food grade quality, as is the case with Neosepta® at least in Europe. Neosepta® membranes have further advantages well known by the specialist, such as low electric resistance, low diffusion coefficients, high mechanical strength, high dimensional stability and high resistance to chemical attack.
In a particular embodiment, the electrodialysis stack contains at least five alternating pairs of cation selective membranes and anion selective membranes, particularly at least 10 alternating pairs.

The active part of the stack is composed of the same number of anion and cation selective membranes but the proportion of ASG and AMS, and the proportion of CSG and CMS is variable and is adjusted advantageously to the composition of feed water. Unexpectedly, the inventors have discovered that by choosing precisely the proportion of each type of membrane, it is possible to achieve a substantially similar rate of removal for each valuable ion present in the raw water of RO step. The inventors have also unexpectedly discovered that the order in which the membranes are stacked up has no influence on the removal rate of each ion, as long as end membranes are cation selective and each anion selective membrane is comprised between 2 cation selective membranes. It is understood that the required proportion of ASG/AMS and CSG/CMS is obtained in a single stack.
For each valuable dissolved mineral ion, this removal rate, or dilution factor (DFᵢ), is calculated to be the ratio of concentration of the ion in the feed of ED to the concentration of the same ion in the diluate of ED. This dilution factor is advantageously equal to the concentration factor (CF) obtained with the RO step (equal to the ratio of feed flow of RO over retentate flow of RO), in order to achieve a substantially unchanged content of valuable mineral ions in the final product. Therefore the DFᵢ for each valuable dissolved mineral ion is close enough to CF to achieve a final weight content of valuable dissolved mineral ions in the range 70 to 130% of initial weight content in raw water and more advantageously 80 to 120 %. Even more advantageously, the final range is 90 to 110%, which means no change at all in the composition of valuable dissolved mineral ions, when taking into account the natural variability of a water source and the analytical uncertainties which all account for a ± 10 % of variability in the composition of the raw water feeding the process.
The removal of OSA by ED step is not significant, in order to achieve a final content of OSA in the diluate of ED equivalent to the content of OSA in the feed of RO multiplied by the concentration factor of RO. More advantageously, the loss of silica in the ED step is less than 10 %, particularly less than 5 %.

The electrodialysis initial concentrate solution can be prepared by dissolving any of the following water soluble mineral salts : NaCl, KCl, Na₂SO₄, K₂SO₄, NaNO₃, KNO₃, NaHCO₃, KHCO₃, CaCl₂, MgCl₂ or MgSO₄ in RO permeate water. The solution can be a pure solution of a single anion and a single cation obtained by dissolving only one mineral salt, or a mix of anions and cations obtained by dissolving together several mineral salts of the list. The mineral salt solution is chosen advantageously to fit with the feed water composition, in order to further refine the DFᵢ of each ion. The inventors have showed that by choosing precisely the mineral salt solution, it is possible to further refine the DFᵢ.. In a particular embodiment, the mineral salt solution is chosen between a NaCl solution, a KCl solution, a K₂SO₄ solution and a mixture thereof, advantageously the mineral salt solution is chosen between a KCl solution, a K₂SO₄ solution and a mixture thereof, more advantageously the mineral salt solution is a mix of KCl and K₂SO₄ solution.
The conductivity of the initial concentrate solution of ED is comprised between 0.5 and 5 mS/cm and more advantageously between 1 and 3 mS/cm. During ED, the conductivity of concentrate solution increases due to ions transfer from the diluate compartment. Thus, the conductivity of concentrate compartment is kept constant by purging periodically or continuously a fraction of the concentrate solution and adding fresh water. In a preferred embodiment, the concentrate solution is managed in a feed and bleed mode of functioning, by adding permeate water from the RO, or diluate from ED outlet. This added water can be supplemented with one or more mineral salts before feeding the concentrate compartment, chosen among NaCl, KCl, Na₂SO₄, K₂SO₄, NaNO₃, KNO₃, NaHCO₃, KHCO₃, CaCl₂, MgCl₂ or MgSO₄.
In another mode of functioning of the ED step, a continuous self-cleaning of electrodialysis process is operated by means of periodic reversal of the DC polarity, switching concentrate and diluate flow streams.
The process of the invention can be carried out in continuous or in a batch mode. In a particular embodiment, the two successive steps a) and b) of the process according to the present invention are repeated on the water obtained after step b) as often as necessary in order to obtain a final water with the desired orthosilicic acid and dissolved valuable mineral weight contents, in particular one or two times, more particularly once.
The different passages allow the increase of the final content of orthosilicic acid in the water while avoiding the precipitation of insoluble minerals in the reverse osmosis step.

Advantageously, the retentate obtained after the first step a) of reverse osmosis contains only half of the desired orthosilicic acid weight content of the final water. In a specific embodiment, the purge of the concentrate compartment of ED (C) is mixed with permeate water of RO (P) in order to produce a drinking water (DW) similar to the initial feed water (F) in term of valuable dissolved mineral ion composition and taste, but containing almost no silicon. All the silicon contained in the initial feed raw water (F) is recovered in the diluate of ED (D), which contains also valuable dissolved mineral ions at a concentration similar to that of initial feed water (F). This is illustrated in Figure 2.

In another particular embodiment, the effluent from the concentrating compartment of the electrodialysis stack is added to the permeate of reverse osmosis in order to form mineral drinking water.

In another particular embodiment, the weight content of nitrate ion in final water is at least less than 80% of the content in the raw water, particularly less than 70%, more particularly less than 50%.
The inventors also unexpectedly discovered that, while achieving a final content of valuable dissolved mineral ions mostly unchanged, it is possible to get an enhanced removal of undesirable nitrate ion, nitrate ion having been associated to adverse health effects due to its conversion into nitrite and nitrosamine in human body, that are know to be carcinogenic substances.

The present invention also concerns a still drinking water characterized in that it contains a sodium dissolved content of less than 20 mg/L, an orthosilicic acid content comprised between 75 and 120 mg/L of SiO₂, and a dry residue between 400 and 1100mg/L.
The still drinking water is calories free, preservatives free, allergen free and has a neutral pH. More advantageously, it does not contain any organic molecules. Advantageously the orthosilicic acid contained therein is of natural origin, coming from the initial water and not from an exogenous source.
Advantageously, the still drinking water is obtainable by the process according to the present invention.
More advantageously, the still drinking water according to the present invention contains less than 10mg/L of dissolved nitrate ions.
The present invention also concerns the use of the still drinking water according to the present invention or obtained by the process according to the present invention for strengthening and/or beautifying the skin, the hair and the nails and/or for the prevention of wrinkles and/or for the prevention of skin dehydration.
Finally, the present invention concerns the still drinking water according to the present invention or obtained by the process according to the present invention for use as drug, in particular for the treatment and/or prevention of osteoporosis, of aluminum toxicity, for the prevention of neurodegenerative diseases, in particular Alzheimer's disease, and/or for the prevention of cardiovascular diseases and/or atherosclerosis, and/or for the prevention of skin diseases and/or for the prevention and/or treatment of hypertension.
Because of the presence of high level of OSA in the water according to the present invention, together with other valuable minerals, the still water according to the present invention has a positive effects on force and brightness of hairs, on strength and elasticity of skin, hydration keeping and anti-inflammatory rule on skin, on the protection against the skin diseases, in the collagen synthesis and therefore the prevention of wrinkles, on strength and amelioration of the nails quality and on better protection against nail infections. It can also be used in the treatment for gastric hyper-acidity. Since silicon and calcium are essential to the formation of bone matrix and bone mineralization, the still water according to the present invention can be used in that regards, and in particular for increasing bone volume in women suffering from osteoporosis or having a bone weight deficit. The still water according to the present invention can also be used for preventing aluminium toxicity by sequestration of aluminium by the silicon and therefore for the prevention of cognitive impairments and neurodegenerative diseases such as Alzheimer's disease.
The characteristics of the present invention will be further explained in the examples below.

### FIGURES

**Figure 1:** Figure 1 represents possible configurations of the reverse osmosis step
**Figure 2:** Figure 2 represents a specific embodiment consisting in mixing permeate of reverse osmosis step with concentrate of electrodialysis step
**Figure 3:** Figure 3 represents a mix of AMS / ASG / CMS / CSG membranes stacked up in the electrodialysis device used in Examples 3 to 7 and 11 and 12.
**Figure 4:** Figure 4 represents the removal C/Co (expressed in %) achieved for each dissolved mineral ion as a function of ED time in Example 3. (C = final concentration of the ion; C₀ = concentration of the ion in the feed water of RO)
**Figure 5:** Figure 5 represents the removal C/Co (expressed in %) achieved for each dissolved mineral ion as a function of ED time in Example 4. (C = final concentration of the ion; C₀ = concentration of the ion in the feed water of RO)
**Figure 6:** Figure 6 represents the removal C/Co (expressed in %) achieved for each dissolved mineral ion as a function of ED time in Example 5. (C = final concentration of the ion; C₀ = concentration of the ion in the feed water of RO)
**Figure 7:** Figure 7 represents the removal C/Co (expressed in %) achieved for each dissolved mineral ion as a function of ED time in Example 6. (C = final concentration of the ion; C₀ = concentration of the ion in the feed water of RO)
**Figure 8:** Figure 8 represents the removal C/Co (expressed in %) achieved for each dissolved mineral ion as a function of ED time in Example 7. (C = final concentration of the ion; C₀ = concentration of the ion in the feed water of RO).
**Figure 9:** Figure 9 represents the removal C/Co (expressed in %) achieved for each dissolved mineral ion as a function of ED time in Example 11. (C = final concentration of the ion; C₀ = concentration of the ion in the feed water of RO).
**Figure 10:** Figure 10 represents the removal C/Co (expressed in %) achieved for each dissolved mineral ion as a function of ED time in Example 12 (C = final concentration of the ion; C₀ = concentration of the ion in the feed water of RO).

### EXAMPLES

### Example 1

Two commercial RO modules BW30-2540 and SW30-2540 from Dow Filmtec (The Dow Chemical Company, Midland, MI) mounted in a stainless steel pressure vessel were used in this test. The configuration of the system was as illustrated in Figure 1 B: 1 pass & 1 stage of RO with recirculation of part of the retentate to the feed. The system was run in continuous mode with a feed water A, having the characteristics reported in Table 1:

**Table 1: Characteristics of feed water A**

| **Temperature** | | | **pH** | | | **Conductivity** | | | | **Dry residue** | | |
|---|---|---|---|---|---|---|---|---|---|---|---|---|
| 15 ± 1°C | | | 6.3 | | | 257 µS/cm | | | | 168 mg/l | | |

| **(composition in mg/l)** | | | | | | | | | | | | |
|---|---|---|---|---|---|---|---|---|---|---|---|---|
| **Ca²⁺** | **Mg²⁺** | **Na⁺** | **K⁺** | **HCO₃⁻** | **Cl⁻** | **SO₄²⁻** | **NO₃** | **PO₄³** | **F⁻** | **Ba²⁺** | **Sr²⁺** | **OSA** |
| 10.6 | 8.2 | 26.1 | 6.2 | 109 | 13.0 | 13.8 | 3.5 | 0.60 | 0.00 | 0.001 | 0.002 | 32.0 |

During the 1^{st} part of the test, the BW30-2540 membrane was mounted in the pressure vessel. ROSA v6.1.5 free software from Filmtec was used prior to the test, to simulate the RO system and define the process parameters to achieve a CF of 2. The simulation also confirmed no solubility issues in the retentate via a scaling calculation.
When the steady state was reached, the feed flow was 200 1/h and the feed-side pressure of RO was 23 bars. The flow of recirculated retentate was 1366 1/h and the flow of rejected retentate was 100 1/h. The conductivity of the retentate was 503 µS/cm. The flow of rejected permeate was 100 1/h and its conductivity was 8.5 µS/cm. The mineral ion composition of retentate and permeate are reported in Table 2. For each mineral ion, the recovery rate in the retentate over a 1 hour time period was calculated and is also reported in Table 2.

**Table 2: Composition of permeate and retentate at steady state and recovery rate of each mineral ion in the retentate with RO module BW30-2540**

| | **Ca²⁺** | **Mg²⁺** | **Na⁺** | **K⁺** | **HCO₃⁻** | **Cl⁻** | **SO₄²⁻** | **NO₃⁻** | **OSA** |
|---|---|---|---|---|---|---|---|---|---|
| Permeate (mg/l) | 0.2 | 0.1 | 1.5 | 0.7 | 4.9 | 0.3 | 0.0 | 0.0 | 0.5 |
| Retentate (mg/l) | 22.7 | 17.2 | 55.2 | 12.5 | 225.7 | 26.5 | 29.5 | 8.5 | 65 |
| Recovery in retentate (%) | 95 | 104 | 104 | 99 | 96 | 101 | 98 | 94 | 95 |

The recovery rate of each valuable dissolved mineral ion Ca²⁺, Mg²⁺, Na⁺, K⁺, HCO₃⁻, Cl⁻, SO₄²⁻ and orthosilicic acid is comprised in the range 95 to 105%. A recovery rate above 100% for some mineral ions is not surprising, and is related to the analytical margin of error.
From this test it can be clearly observed that 100 ± 5 % of all dissolved valuable mineral ions and orthosilicic acid are recovered in the retentate when a CF = 2 is operated.

During the 2^{nd} part of the test, the SW30-2540 membrane was mounted in the pressure vessel. ROSA v6.1.5 was again used prior to the test, to simulate the RO system and define the process parameters to achieve a CF of 2. The simulation also confirmed no solubility issues in the retentate via a scaling calculation. When the steady state was reached, the feed flow was 204 1/h and the feed-side pressure of RO was 35 bars. The flow of recirculated retentate was 800 1/h and the flow of rejected retentate was 102 1/h. The conductivity of the retentate was 517 µS/cm. The flow of rejected permeate was 102 1/h and its conductivity was 3.2 µS/cm. The mineral ion composition of retentate and permeate are reported in Table 3. For each mineral ion, the recovery rate in the retentate over a 1 hour time period was calculated and is also reported in Table 3.

**Table 3: Composition of permeate and retentate at steady state and recovery rate of each mineral ion in the retentate with RO module SW30-2540**

| | **Ca²⁺** | **Mg²⁺** | **Na⁺** | **K⁺** | **HCO₃⁻** | **Cl⁻** | **SO₄²⁻** | **NO₃⁻** | **OSA** |
|---|---|---|---|---|---|---|---|---|---|
| Permeate (mg/l) | 0.0 | 0.0 | 0.4 | 0.0 | 3.1 | 0.0 | 0.0 | 0.0 | 0.2 |
| Retentate (mg/l) | 24.6 | 17.2 | 55.6 | 12.1 | 235.5 | 28.3 | 30.3 | 7.4 | 67.0 |
| Recovery in retentate (%) | 101 | 101 | 102 | 94 | 98 | 105 | 99 | 90 | 96 |

The recovery rate of each valuable dissolved mineral ion Ca²⁺, Mg²⁺, Na⁺, K⁺, HCO₃⁻, Cl⁻, SO₄²⁻ and orthosilicic acid is comprised in the range 94 to 105%. Results with modules BW30-2540 and SW30-2540 are very similar. However, BW module requires a lower pressure to achieve CF = 2, which in turn requires less energy for the pump.

### Example 2

The test was performed with same equipment and same configuration as in Example 1, but only BW30-2540 RO module was used. The system was run in continuous mode with a feed water B, different from feed water A, having the characteristics reported in Table 4:

**Table 4: Characteristics of feed water B**

| **Temperature** | | | **pH** | | | **Conductivity** | | | | **Dry residue** | | |
|---|---|---|---|---|---|---|---|---|---|---|---|---|
| 14 ± 1°C | | | 6.7 | | | 196 µS/cm | | | | 129 mg/l | | |

| **(composition in mg/l)** | | | | | | | | | | | | |
|---|---|---|---|---|---|---|---|---|---|---|---|---|
| **Ca²⁺** | **Mg²⁺** | **Na⁺** | **K⁺** | **HCO₃⁻** | **Cl⁻** | **SO₄²⁻** | **NO₃⁻** | **PO₄³⁻** | **F⁻** | **Ba²⁺** | **Sr²⁺** | **OSA** |
| 11.2 | 7.8 | 10.7 | 5.7 | 72 | 13.7 | 6.6 | 6.6 | 0.49 | 0.23 | 0.0004 | 0.059 | 31.0 |

During the 1^{st} part of the test, ROSA v6.1.5 was used to simulate the RO system and define the process parameters to achieve a CF of 2 with feed water B. The simulation also confirmed no solubility issues in the retentate.
When the steady state was reached, the feed flow was 200 1/h and the feed-side pressure of RO was 25 bars. The flow of recirculated retentate was 700 1/h and the flow of rejected retentate was 100 1/h. The conductivity of the retentate was 365 µS/cm. The flow of rejected permeate was 100 1/h and its conductivity was 5.7 µS/cm. The mineral ion composition of retentate and permeate are reported in Table 5. For each mineral ion, the recovery rate in the retentate over a 1 hour time period was calculated and is also reported in Table 5.

**Table 5: Composition of permeate and retentate at steady state and recovery rate of each mineral ion in the retentate with RO module BW30-2540 and CF = 2**

| | **Ca²⁺** | **Mg²⁺** | **Na⁺** | **K⁺** | **HCO₃⁻** | **Cl⁻** | **SO₄²⁻** | **NO₃⁻** | **F⁻** | **OSA** |
|---|---|---|---|---|---|---|---|---|---|---|
| Permeate (mg/l) | 0.0 | 0.0 | 0.7 | 0.3 | 3.0 | 0.2 | 0.0 | 1.0 | 0.0 | 0.5 |
| Retentate (mg/l) | 21.1 | 14.5 | 20.4 | 10 | 138 | 27.5 | 13.5 | 13.2 | 0.46 | 58.1 |
| Recovery in retentate (%) | 95 | 96 | 95 | 95 | 96 | 101 | 101 | 99 | 93 | 93 |

The recovery rate of each valuable dissolved mineral ion Ca²⁺, Mg²⁺, Na⁺, K⁺, HCO₃⁻, Cl⁻, SO₄²⁻ and orthosilicic acid is comprised in the range 93 to 101% with water B, different from water A.

During the 2^{nd} part of the test, ROSA v6.1.5 was used to simulate the RO system and define the process parameters to achieve a CF of 3 with feed water B. The simulation also confirmed no solubility issues in the retentate.
When the steady state was reached, the feed flow was 150 1/h and the feed-side pressure of RO was 24 bars. The flow of recirculated retentate was 750 1/h and the flow of rejected retentate was 50 l/h. The conductivity of the retentate was 535 µS/cm. The flow of rejected permeate was 100 1/h and its conductivity was 5.2 µS/cm. The mineral ion composition of retentate and permeate are reported in Table 6. For each mineral ion, the recovery rate in the retentate over a 1 hour time period was calculated and is also reported in Table 6.

**Table 6: Composition of permeate and retentate at steady state and recovery rate of each mineral ion in the retentate with RO module BW30-2540 and CF = 3**

| | **Ca²⁺** | **Mg²⁺** | **Na⁺** | **K⁺** | **HCO₃⁻** | **Cl⁻** | **SO₄²⁻** | **NO₃⁻** | **F⁻** | **OSA** |
|---|---|---|---|---|---|---|---|---|---|---|
| Permeate (mg/l) | 0.0 | 0.0 | 0.6 | 0.25 | 2.5 | 0.2 | 0.0 | 0.3 | 0.0 | 0.5 |
| Retentate (mg/l) | 31.5 | 22.3 | 29.3 | 16.8 | 206.9 | 43.2 | 21.6 | 19.6 | 0.8 | 85.5 |
| Recovery in retentate (%) | 93 | 93 | 91 | 92 | 95 | 104 | 111 | 101 | 121 | 91 |

The recovery rate of each valuable dissolved mineral ion Ca²⁺, Mg²⁺, Na⁺, K⁺, HCO₃⁻, Cl⁻, SO₄²⁻ and orthosilicic acid is comprised in the range 93 to 111% with water B, thus achieving a CF = 3 with no substantial loss of valuable mineral ions in the retentate.

### Example 3

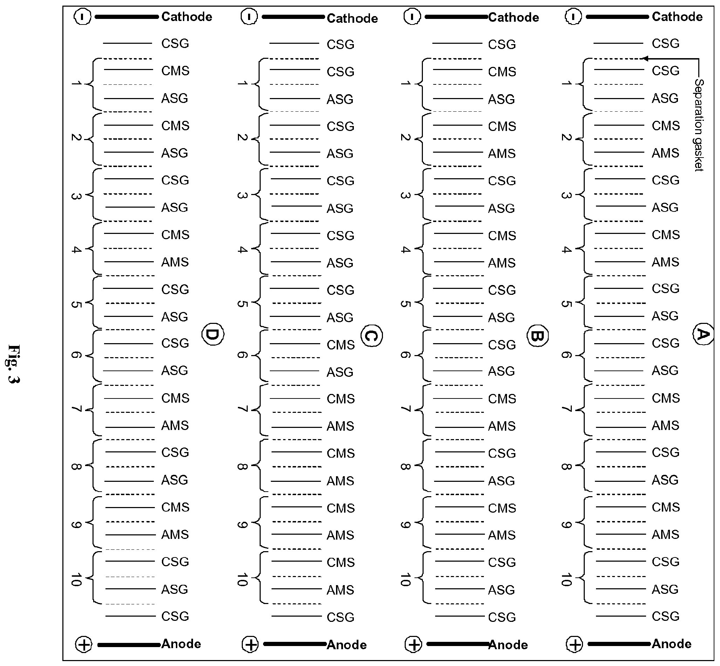

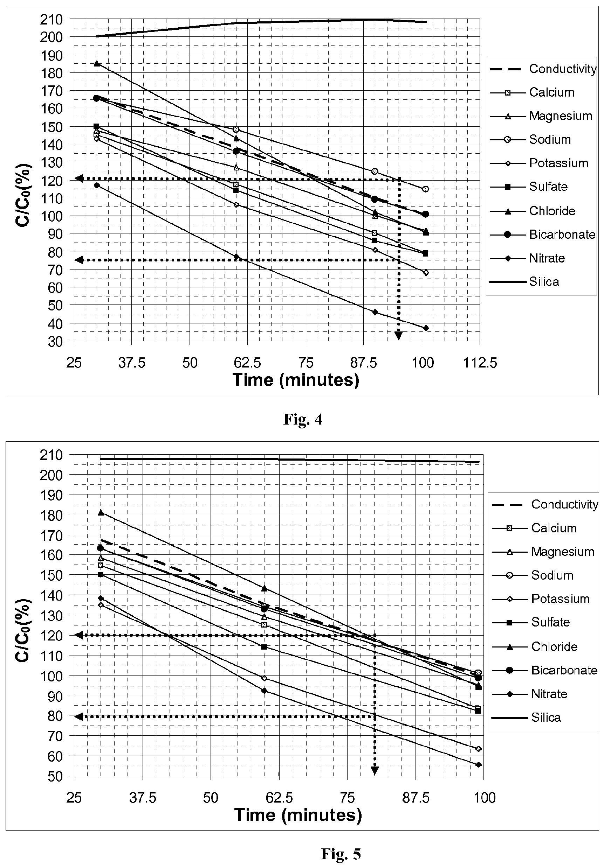

30 liters of retentate produced according to example 1 and feed water A is used in this test. The water is processed by an electrodialysis device EUR2C-10P (EURODIA Industries, Rungis, France) in batch mode. The electrodialysis stack is composed of 10 pairs of Neosepta® anion and cation selective membranes, placed alternatively in a clamping system similar to a filter press and forming 10 active cells. One cation selective membranes CSG is added at each extremity of the electrodialysis stack in order to isolate the electrolyte. The active surface of each cell is 0.02 m² and the total active surface of the stack is 0.2 m². Each electrodialysis cell, having a dilution and a concentration compartment, is made of:
- A separation gasket, equipped with a netting that promotes turbulence,
- An anion selective membrane,
- A separation gasket,
- A cation selective membrane,
- A separation gasket.
The mix of AMS / ASG / CMS / CSG membranes in the stack, excluding the extremity cationic membranes, is composed of 4 CMS, 6 CSG, 4 AMS and 6 ASG and is stacked up as illustrated in Figure 3A. The electrodes that are placed at the end of the device are a Titanium - Platinum anode and a stainless steel cathode. They are flushed by an electrolyte made of a NaNO₃ solution with a conductivity of 19.4 mS/cm at 20°C and a pH of 6.5.
The test is performed in a constant voltage/variable current mode, in which a desired voltage of 8 V is set and the current is allowed to automatically adjust to maintain the voltage and is not allowed to approach the limiting current. The temperature is kept constant at 19.5 ± 0.6°C by recirculating the diluate in a tank equipped with a cooling water jacket, in order to compensate the heating produced by the ED.
In this test, the initial concentrate solution was a NaCl solution with a conductivity of 1.0 mS/cm at 20°C and a pH of 7.7. The conductivity of the concentrate was kept in the range 1.1 ∼ 1.6 mS/cm by periodically purging the concentrate and adding permeate water from RO.
The composition of the diluate is measured every 30 minutes and at the end of the test, when 100% of the conductivity of the feed water of RO is reached and is illustrated in Figure 4. In Figure 4 is also reported the removal for silicon and for conductivity. From this Figure it can be clearly observed that after 95 minutes, the composition of Ca²⁺, Mg²⁺, Na⁺, K⁺, Cl⁻, SO₄²⁻, and HCO₃⁻ is in the range 75 to 120%, while silicon content (expressed as SiO₂) remains unchanged at 208%. Undesirable NO₃⁻ ion is removed down to 42% of the initial content. The final water composition is reported in Table 7.

**Table 7: Final water composition**

| | |
|---|---|
| Calcium (mg/l) | 9.0 |
| Magnesium (mg/l) | 7.9 |
| Sodium (mg/l) | 31.3 |
| Potassium (mg/l) | 4.7 |
| Bicarbonate (mg/l) | 114 |
| Chloride (mg/l) | 12.6 |
| Sulfate (mg/l) | 11.5 |
| Nitrate (mg/l) | 1.5 |
| OSA (mg/l SiO₂) | 66.9 |
| Dry Residue (mg/l) | 202 |

### Example 4

The example 3 is repeated, but the first CMS membrane after the cathode is replaced by a CSG membrane as illustrated in Figure 3B, in order to narrow the removal rate of each valuable dissolved mineral ion. The electrodes are flushed by an electrolyte made of a NaNO₃ solution with a conductivity of 19.7 mS/cm at 20°C and a pH of 9.9.The test is performed in a constant voltage/variable current mode, in which a desired voltage of 8 V is set and the current is allowed to automatically adjust to maintain the voltage and is not allowed to approach the limiting current. The temperature is kept constant at 19.4 ± 0.6°C by recirculating the diluate in a tank equipped with a "double envelope". Inside "double envelope" a thermal regulating fluid is circulated in order to compensate the heating produced by the ED. In this test, the initial concentrate solution was a NaCl solution with a conductivity of 1.1 mS/cm at 20°C and a pH of 7.3. The conductivity of the concentrate was kept in the range 1.1 ∼ 1.9 mS/cm by periodically purging the concentrate and adding permeate water from RO. The composition of the diluate is measured every 30 minutes and at the end of the test, when 100% of the conductivity of the feed water of RO is reached and is illustrated in Figure 5. In Figure 5 is also reported the removal for silicon and for conductivity. From this Figure it can be clearly observed that after 80 minutes, the composition of Ca²⁺, Mg²⁺, Na⁺, K⁺, Cl⁻, SO₄²⁻, and HCO₃⁻ is in the more restricted range 80 to 120%, while silicon content (expressed as SiO₂) remains unchanged at 206%. Undesirable NO₃⁻ ion is removed down to 73% of the initial content. The final water composition is reported in Table 8.

**Table 8: final water composition**

| | |
|---|---|
| Calcium (mg/l) | 11.0 |
| Magnesium (mg/l) | 9.2 |
| Sodium (mg/l) | 30.5 |
| Potassium (mg/l) | 5.1 |
| Bicarbonate (mg/l) | 126 |
| Chloride (mg/l) | 15.4 |
| Sulfate (mg/l) | 13.4 |
| Nitrate (mg/l) | 2.6 |
| OSA (mg/l SiO₂) | 65.9 |
| Dry Residue (mg/l) | 216 |

### Example 5

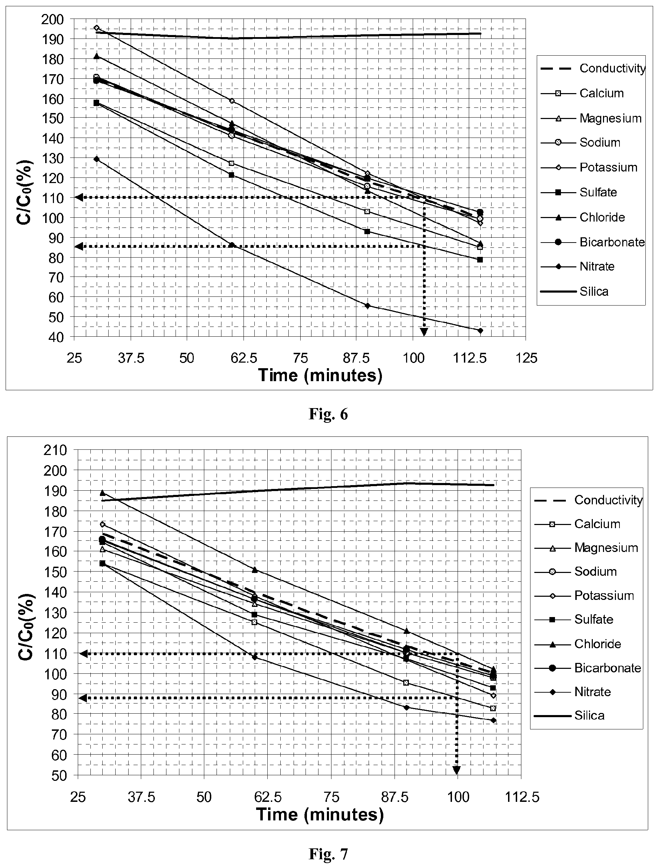

The example 4 is repeated but the initial concentrate solution of NaCl is replaced by a KCl solution with a conductivity of 1.0 mS/cm at 20°C and a pH of 6.3. The conductivity of the concentrate is kept in the range 1.1 ∼ 1.5 mS/cm by periodically purging the concentrate and adding permeate water from RO. The temperature is kept constant at 20.8 ± 0.3 °C.
The composition of the diluate is measured every 30 minutes and at the end of the test, when 100% of the conductivity of the feed water of RO is reached and is illustrated in Figure 6. In Figure 6 is also reported the removal for silicon and for conductivity. From this Figure it can be clearly observed that after 99 minutes, the composition of Ca²⁺, Mg²⁺, Na⁺, K⁺, Cl⁻, SO₄²⁻, and HCO₃⁻ is in the range 85 to 110%, while silicon content (expressed as SiO₂) remains unchanged at 193%. The nitrate is removed down to 49% of its initial content. The final water composition is reported in Table 9. This example illustrates that by replacing Na by K in the initial concentrate, it is possible to reduce the K removal by ED and narrow the removal rate of each valuable dissolved mineral ion in order to achieve a final concentration in the diluate (C) closer to 100 % of the concentration in the feed water of RO (C₀).

**Table 9: final water composition**

| | |
|---|---|
| Calcium (mg/l) | 9.9 |
| Magnesium (mg/l) | 9.0 |
| Sodium (mg/l) | 27.9 |
| Potassium (mg/l) | 6.8 |
| Bicarbonate (mg/l) | 120 |
| Chloride (mg/l) | 13.0 |
| Sulfate (mg/l) | 11.9 |
| Nitrate (mg/l) | 1.7 |
| OSA (mg/l SiO₂) | 61.4 |
| Dry Residue (mg/l) | 202 |

### Example 6

Example 5 is repeated but the ED membranes are stacked differently (Figure 3 C), in order to demonstrate that the choice of the membranes is important, not how they are stacked. For this test, the initial KCl concentrate solution has a conductivity of 1.0 mS/cm at 19°C and a pH of 7.8. The conductivity of the concentrate is kept in the range 1.1 ∼ 1.5 mS/cm by periodically purging the concentrate and adding permeate water from RO. The temperature is kept constant at 21.1 ± 0.3 °C.
The composition of the diluate is measured every 30 minutes and at the end of the test, when 100% of the conductivity of the feed water of RO is reached and is illustrated in Figure 7. In Figure 7 is also reported the removal for silicon and for conductivity. From this Figure it can be clearly observed that after 100 minutes, the composition of Ca²⁺, Mg²⁺, Na⁺, K⁺, Cl⁻, SO₄²⁻, and HCO₃⁻ is in the range 88 to 110%, while silicon content (expressed as SiO₂) remains unchanged at 193%. These results are comparable to those illustrated in Figure 6. The final water composition is reported in Table 10.

**Table 10: final water composition**

| | |
|---|---|
| Calcium (mg/l) | 10.3 |
| Magnesium (mg/l) | 8.4 |
| Sodium (mg/l) | 26.8 |
| Potassium (mg/l) | 6.1 |
| Bicarbonate (mg/l) | 113 |
| Chloride (mg/l) | 14.3 |
| Sulfate (mg/l) | 13.7 |
| Nitrate (mg/l) | 2.8 |
| OSA (mg/l SiO₂) | 61.7 |
| Dry Residue (mg/l) | 201 |

### Example 7

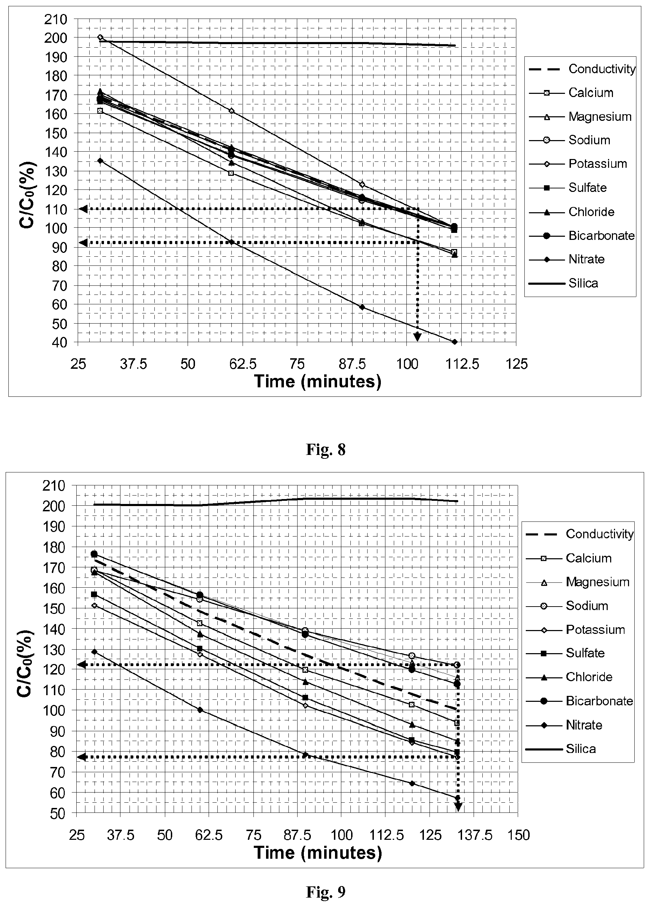

The example 6 is repeated, with the same membrane stacking as illustrated in Figure 3C, but the initial concentrate solution of NaCl is replaced by a mix of KCl and K₂SO₄ solution with a conductivity of 1.0 mS/cm at 20°C and a pH of 7.1. 2/3 of this conductivity are obtained by KCl and 1/3 by K₂SO₄. The conductivity of the concentrate is kept in the range 1.1 ∼ 1.5 mS/cm by periodically purging the concentrate and adding permeate water from RO. The temperature is kept constant at 21.6 ± 0.3 °C.
The composition of the diluate is measured every 30 minutes and at the end of the test, when 100% of the conductivity of the feed water of RO is reached and is illustrated in Figure 8. In Figure 8 is also reported the removal for silicon and for conductivity. From this Figure it can be clearly observed that after 102 minutes, the composition of Ca²⁺, Mg²⁺, Na⁺, K⁺, Cl⁻, SO₄²⁻, and HCO₃⁻ is in the range 92 to 110%, while silicon content (expressed as SiO₂) remains unchanged at 196%. The nitrate is removed down to 47% of its initial content. The final water composition is reported in Table 11. This example illustrates that by replacing K⁺ and Cl⁻ by a mix of K⁺, Cl⁻ and SO₄²⁻ in the initial concentrate, it is possible to further narrow the removal rate of each valuable dissolved mineral ion in order to achieve a final concentration in the diluate (C) closer to 100 % of the concentration in the feed water of RO (C₀).

**Table 11: final water composition**

| | |
|---|---|
| Calcium (mg/l) | 9.8 |
| Magnesium (mg/l) | 8.8 |
| Sodium (mg/l) | 27.9 |
| Potassium (mg/l) | 6.8 |
| Bicarbonate (mg/l) | 117 |
| Chloride (mg/l) | 12.1 |
| Sulfate (mg/l) | 14.5 |
| Nitrate (mg/l) | 1.6 |
| OSA (mg/l SiO₂) | 62.7 |
| Dry Residue (mg/l) | 203 |

### Example 8

Stability of drinking water fortified with OSA could be an issue if that water is stored at low temperature, for example in a refrigerator at 4°C. In fact, solubility of orthosilicic acid in water decreases with decreasing temperature. The solubility is 115 mg/l at 20°C and a pH of 7.0, but it's only 85 mg/l at 4°C and same pH. Below the limit of solubility, colloidal silica is formed.
Water fortified with OSA at 100 mg/l was stored in crown cap closed 1 liter glass bottles during several days at 4°C. A bottle was picked-up from time to time, opened and half of the volume was filtered immediately with 0.45 µm filter, in order to remove from the filtered water the colloidal silica potentially formed during storage at 4°C. The filtered and non filtered water from the same bottle where analysed for dissolved silicon content by using the Molibdate colorimetric method. As shown in Table 12, the silicon was totally in the soluble orthosilicic acid form, even after 3 month at 4°C, thus demonstrating that the fortified drinking water is perfectly storage stable.

**Table 12: Weight percent of soluble non filterable silicon content (OSA) of the water after storage at the temperature of 4°C**

| Storage time at 4°C (days) | Dissolved non filtrable silicon content (%) |
|---|---|
| 14 | 100 |
| 21 | 100 |
| 28 | 100 |
| 56 | 100 |
| 90 | 100 |

### Comparative example 9

Example 9 is an illustrative example of the impossibility to achieve the required removal rate for all valuable dissolved mineral ions with a non-mixed membrane stack. In this example, the ED device described in example 3 was used, and all membranes were AMS and CMS except 2 CSG at the extremity to isolate the electrolyte. In this test, the initial concentrate solution was a NaCl solution with a conductivity of 1.4 mS/cm at 20°C and a pH of 7.8. The composition of the diluate was measured when 100% of the conductivity of the feed water of RO (Water A) was reached. The following Table 13 illustrates the removal achieved for each dissolved mineral ion and is expressed in %. From this table it can be clearly observed that none of the dissolved ions was in the target range of 70 to 130%, except bicarbonate. However nitrate was well removed and OSA was not removed at all.

**Table 13: % of removal for each dissolved ions in the final water**

| | (%) |
|---|---|
| Conductivity | 100 |
| Calcium | 133 |
| Magnesium | 166 |
| Sodium | 57 |
| Potassium | 42 |
| Bicarbonate | 98 |
| Chloride | 48 |
| Sulfate | 152 |
| Nitrate | 33 |
| OSA | 200 |

### Example 10

The test was performed with same equipment and same configuration as in Example 1, but HSRO-4040 module was used, consisting in hot sanitizable membranes from Dow Filmtec. The system was run in continuous mode with a feed water C, having the characteristics reported in Table 14.

**Table 14: Characteristics of feed water C**

| **Temperature** | | | **pH** | | | **Conductivity** | | | | **Dry residue** | | |
|---|---|---|---|---|---|---|---|---|---|---|---|---|
| 14 ± 1°C | | | 5.97 | | | 478 µS/cm | | | | 288 mg/l | | |
| **(composition in mg/l)** | | | | | | | | | | | | |
| **Ca²⁺** | **Mg²⁺** | **Na⁺** | **K ⁺** | **HCO₃⁻** | **Cl⁻** | **SO ₄²⁻** | **NO₃⁻** | **PO₄³⁻** | **F⁻** | **Ba²⁺** | **Sr²⁺** | **OSA** |
| 36.5 | 13.8 | 9.1 | 3 7.9 | 151.9 | 43.0 | 41.5 | 7.0 | 0.44 | 0.18 | 0.0004 | 0.062 | 22.9 |

Before starting the test, ROSA v7.0.0 was used to simulate the RO system and define the process parameters to achieve a CF of 2. The simulation also confirmed no solubility issues in the retentate.
When the steady state was reached, the feed flow was 396 1/h and the feed-side pressure of RO was 7.2 bars. The flow of recirculated retentate was 1300 1/h and the flow of rejected retentate was 198 1/h. The conductivity of the retentate was 902 µS/cm. The flow of rejected permeate was 200 1/h and its conductivity was 24.2 µS/cm. The mineral ion composition of retentate and permeate are reported in Table 15. For each mineral ion, the recovery rate in the retentate over a 1 hour time period was calculated and is also reported in Table 15.

**Table 15: Composition of permeate and retentate at steady state and recovery rate of each mineral ion in the retentate with RO module HSRO-4040 and CF = 2**

| | **Ca²⁺** | **Mg²⁺** | **Na⁺** | **K⁺** | **HCO₃⁻** | **Cl⁻** | **SO₄²⁻** | **NO₃⁻** | **F⁻** | **OSA** |
|---|---|---|---|---|---|---|---|---|---|---|
| Permeate (mg/l) | 0.3 | 0.1 | 0.5 | 4.7 | 8.8 | 0.5 | 0.1 | 0.4 | 0.0 | 0.2 |
| Retentate (mg/l) | 73 | 27.6 | 18.2 | 70.6 | 297.6 | 85 | 82 | 12.5 | 0.34 | 46.6 |
| Recovery in retentate (%) | 100 | 100 | 100 | 93 | 98 | 99 | 99 | 89 | 94 | 102 |

The recovery rate of each valuable dissolved mineral ion Ca²⁺, Mg²⁺, Na⁺, K⁺, HCO₃⁻, Cl⁻, SO₄²⁻ and orthosilicic acid is comprised in the range 93 to 102% with water C.
During the 2^{nd} part of the test, ROSA v7.0.0 was used to simulate the RO system and define the process parameters to achieve a CF of 3 with feed water D, very similar to water C, having the characteristics reported in table 16. The simulation also confirmed no solubility issues in the retentate.

**Table 16: Characteristics of feed water D**

| **Temperature** | | | **pH** | | | **Conductivity** | | | | **Dry residue** | | |
|---|---|---|---|---|---|---|---|---|---|---|---|---|
| 18 ± 1°C | | | 5.84 | | | 484 µS/cm | | | | 290 mg/l | | |

| **(composition in mg/1)** | | | | | | | | | | | | |
|---|---|---|---|---|---|---|---|---|---|---|---|---|
| **Ca²⁺** | **Mg²⁺** | **Na⁺** | **K⁺** | **HCO₃⁻** | **Cl⁻** | **SO₄²⁻** | **NO₃⁻** | **PO₄³⁻** | **F⁻** | **Ba²⁺** | **Sr²⁺** | **OSA** |
| 37.2 | 14.1 | 9.0 | 3 9.8 | 155.6 | 42 | 40 | 5.8 | 0.62 | 0.17 | 0.0005 | 0.058 | 23.4 |

When the steady state was reached, the feed flow was 404 1/h and the feed-side pressure of RO was 9 bars. The flow of recirculated retentate was 1300 1/h and the flow of rejected retentate was 140 1/h. The conductivity of the retentate was 1284 µS/cm. The flow of rejected permeate was 257 1/h and its conductivity was 26.4 µS/cm. The mineral ion composition of retentate and permeate are reported in Table 17. For each mineral ion, the recovery rate in the retentate over a 1 hour time period was calculated and is also reported in Table 17.

**Table 17: Composition of permeate and retentate at steady state and recovery rate of each mineral ion in the retentate with RO module HSRO-4040 and CF = 3**

| | **Ca²⁺** | **Mg²⁺** | **Na⁺** | **K⁺** | **HCO₃⁻** | **Cl⁻** | **SO₄²⁻** | **NO₃⁻** | **F⁻** | **OSA** |
|---|---|---|---|---|---|---|---|---|---|---|
| Permeate (mg/l) | 0.2 | 0.0 | 0.6 | 5.4 | 10 | 0.7 | 0.15 | 0.5 | 0.0 | 0.2 |
| Retentate (mg/l) | 111.3 | 42 | 26.1 | 108.3 | 445.4 | 123 | 118 | 16.5 | 0.5 | 69.8 |
| Recovery in retentate (%) | 104 | 103 | 100 | 94 | 99 | 101 | 102 | 99 | 106 | 103 |

The recovery rate of each valuable dissolved mineral ion Ca²⁺, Mg²⁺, Na⁺, K⁺, HCO₃⁻, Cl⁻, SO₄²⁻ and orthosilicic acid is comprised in the range 94 to 104% with water D, thus achieving a CF = 3 with no substantial loss of valuable mineral ions in the retentate.

### Example 11

The example 4 is repeated, but the first AMS membrane after the cathode is replaced by a ASG membrane as illustrated in Figure 3D, in order to narrow the removal rate of each valuable dissolved mineral ion. The electrodes are flushed by an electrolyte made of a NaNO₃ solution with a conductivity of 19.1 mS/cm at 21.1°C and a pH of 9.5. 30 liters of retentate produced according to example 10 and feed water C (CF = 2) is used in this test.
The test is performed in a constant voltage/variable current mode, in which a desired voltage of 9 V is set and the current is allowed to automatically adjust to maintain the voltage and is not allowed to approach the limiting current. The temperature is kept constant at 18.7 ± 0.7°C.
In this test, the initial concentrate solution is a KCl solution with a conductivity of 1.0 mS/cm at 21.4°C and a pH of 6.7. The conductivity of the concentrate is kept in the range 1.0 ∼ 2.1 mS/cm by periodically purging the concentrate and adding permeate water from RO.
The composition of the diluate is measured every 30 minutes and at the end of the test, when 100% of the conductivity of the feed water of RO is reached (Figure 9). In Figure 9 is also reported the removal for silicon and for conductivity. From this Figure it can be clearly observed that after 133 minutes, the composition of Ca²⁺, Mg²⁺, Na⁺, K⁺, Cl⁻, SO₄²⁻, and HCO₃⁻ is in the range 75 to 125% of their content in water C, while silicon content (expressed as SiO₂) remains unchanged at 202% of content in water C. Undesirable NO₃⁻ ion is removed down to 57% of the initial content. Water obtained by the process contains a sodium dissolved content of 11.1 mg/l, an orthosilicic acid content of 46.3 mg/L of SiO₂, and a dry residue of 296.6 mg/L. The complete final water composition is reported in Table 18.

**Table 18: final water composition**

| | |
|---|---|
| Calcium (mg/l) | 34.3 |
| Magnesium (mg/l) | 16 |
| Sodium (mg/l) | 11.1 |
| Potassium (mg/l) | 29.3 |
| Bicarbonate (mg/l) | 171.1 |
| Chloride (mg/l) | 36.5 |
| Sulfate (mg/l) | 33 |
| Nitrate (mg/l) | 4 |
| OSA (mg/l SiO₂) | 46.3 |
| Dry Residue (mg/l) | 296.6 |

### Example 12

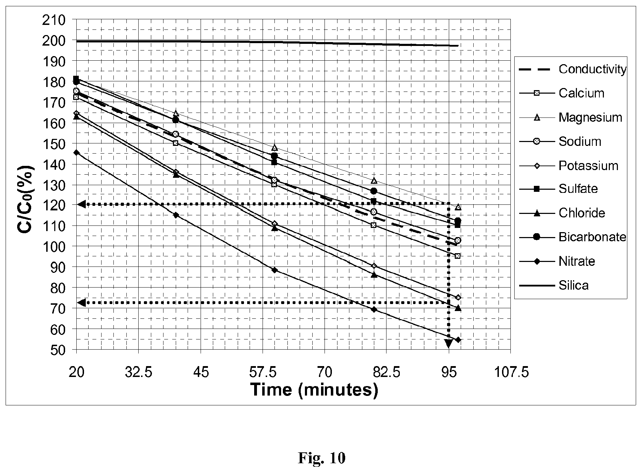

The example 11 is repeated. The electrodes are flushed by an electrolyte made of a NaNO₃ solution with a conductivity of 18.7 mS/cm at 21 °C and a pH of 6.5. 30 liters of retentate produced according to example 10 and feed water D (CF = 3) are used in this test.
The test is performed in a constant voltage/variable current mode, in which a desired voltage of 13 V is set and the current is allowed to automatically adjust to maintain the voltage and is not allowed to approach the limiting current. The temperature is kept constant at 23.2 ± 1.2°C.
In this test, the initial concentrate solution is a KCl solution with a conductivity of 1.0 mS/cm at 21.3°C and a pH of 7.4. The conductivity of the concentrate is kept in the range 1.0 ∼ 2.6 mS/cm by periodically purging the concentrate and adding permeate water from RO.
The composition of the diluate is measured every 20 minutes and at the end of the test, when 100% of the conductivity of the feed water of RO is reached (Figure 10). In Figure 10 is also reported the removal for silicon and for conductivity. From this Figure it can be clearly observed that after 95 minutes, the composition of Ca²⁺, Mg²⁺, Na⁺, K⁺, Cl⁻, SO₄²⁻, and HCO₃⁻ is in the range 70 to 125%, while silicon content (expressed as SiO₂) remains unchanged at 197%. Undesirable NO₃⁻ ion is removed down to 56% of the initial content. Water obtained by the process contains a sodium dissolved content of 13.6 mg/l, an orthosilicic acid content of 68.7 mg/L of SiO₂, and a dry residue of 447 mg/L. The final water composition is reported in Table 19.

**Table 19 : final water composition**

| | |
|---|---|
| Calcium (mg/l) | 53.9 |
| Magnesium (mg/l) | 25.4 |
| Sodium (mg/l) | 13.6 |
| Potassium (mg/l) | 42 |
| Bicarbonate (mg/l) | 253.2 |
| Chloride (mg/l) | 44.2 |
| Sulfate (mg/l) | 66 |
| Nitrate (mg/l) | 4.6 |
| OSA (mg/l SiO₂) | 68.7 |
| Dry Residue (mg/l) | 447 |

## Claims

1. A process for producing water enriched with natural orthosilicic acid, said process comprising at least the following successive steps:
a. a step of reverse osmosis of a raw water containing an amount of orthosilicic acid less than 45 mg/l of SiO₂ in order to obtain a retentate;
b. a step of electrodialysis of the retentate obtained in step a) with an electrodialysis stack comprising alternating pairs of cation selective membranes and anion selective membranes, said cation selective membranes and anion selective membranes being selected in the group of monovalent selective membranes and standard grade membranes such that the stack contains at least one cation standard grade membrane, one cation monovalent selective membrane, one anion standard grade membrane and one anion monovalent selective membrane;
c. a step of recovery of a final water having a orthosilicic acid weight content equal to at least 150%, particularly at least 200%, more particularly at least 300% of the orthosilicic acid weight content of the raw water, and a dissolved valuable mineral ions weight content comprised in the range of 70 to 130%, particularly in the range of 80 to 120%, more particularly in the range of 90 to 110 % of the dissolved valuable mineral ions weight content of the raw water, the valuable mineral ions being chosen from among calcium, magnesium, sodium, potassium, bicarbonate, sulfate and chloride ions.

2. Process according to claim 1, **characterized in that** it comprises a preliminary step α) before step a), consisting in the decontamination of the raw water in order to obtain a decontaminated raw water and **in that** step a) is carried out on the decontaminated raw water.

3. Process according to claim 1 or 2, **characterized in that** the raw water is selected among groundwaters or surface water, more particularly spring water, mineral water, natural mineral water or drinking waters.

4. Process according to any of claims 1 to 3, **characterized in that** the final water has a orthosilicic acid content comprised between 30 and 120 mg/L of SiO₂, particularly between 45 and 120 mg/L, more particularly between 75 and 120 mg/L.

5. Process according to any of claims 1 to 4, **characterized in that** the final water has a dry residue below 1500 mg/l, particularly below 1100mg/L, more particularly below 500mg/L.

6. Process according to any of claims 1 to 5, **characterized in that** the electrodialysis stack contains at least five alternating pairs of cation selective membranes and anion selective membrane, particularly at least 10 alternating pairs.

7. Process according to any of claims 1 to 6, **characterized in that** the final water has a stable orthosilicic acid weight content at storage temperature, even in particular at a temperature below 10°C, more particularly of about 4°C.

8. Process according to any of claims 1 to 7, **characterized in that** it comprises a final step d) after step c), consisting in the addition of the final water to a beverage and/or a syrup and/or a powder, in order to form a beverage enriched with natural bioavailable orthosilicic acid.

9. Process according to any of claims 1 to 8, **characterized in that** the recovery rate of reverse osmosis is adjusted to keep saturation rate of insoluble minerals in the retentate below 100%, particularly below 90%, more particularly below 80%.

10. Process according to any of claims 1 to 9, **characterized in that** the two successive steps a) and b) are repeated on the water obtained after step b) as often as necessary in order to obtain a final water with the desired orthosilicic acid and dissolved valuable mineral weight contents, in particular one or two times, more particularly once.

11. Process according to claim 10, **characterized in that** the retentate obtained after the first step a) of reverse osmosis contains only half of the desired orthosilicic acid weight content of the final water.

12. Process according to any of claims 1 to 11, **characterized in that** the reverse osmosis membranes are selected among hot sanitizable membranes, sea water desalinisation membranes, brackish water membranes, fouling resistant membranes and low energy membranes, in particular among brackish water membranes and sea water desalinisation membranes.

13. Process according to any of claims 1 to 12, **characterized in that** the rejection rate of dissolved mineral ions and orthosilicic acid in the retentate is at least higher than 80%, particularly higher than 90 %, more particularly higher than 95 %.

14. Process according to any of claims 1 to 13, **characterized in that** the effluent from the concentrating compartment of the electrodialysis stack is added to the permeate of reverse osmosis in order to form mineral drinking water.

15. Process according to any of claims 1 to 15, **characterized in that** content of nitrate ion in final water is at least less than 80% of the weight content in the raw water, particularly less than 70%, more particularly less than 50%.

16. Still drinking water obtainable by the process according to any of claims 1 to 15 **characterized in that** it contains a sodium dissolved content of less than 20 mg/L, an orthosilicic acid content comprised between 75 and 120 mg/L of SiO₂, and a dry residue between 400 and 1100mg/L.

17. Still drinking water according to claim 16, **characterized in that** it contains less than 10mg/L of dissolved nitrate ions.

18. Use of the still drinking water according to claim 16 or 17 for strengthening and/or beautifying the skin, the hair and the nails and/or for the prevention of wrinkles and/or for the prevention of skin dehydration.

19. Still drinking water according to claim 16 or 17 for use as drug, in particular for the treatment and/or prevention of osteoporosis, of aluminum toxicity, for the prevention of neurodegenerative diseases, in particular Alzheimer's disease, and/or for the prevention of cardiovascular diseases and/or atherosclerosis, and/or for the prevention of skin diseases and/or for the prevention and/or treatment of hypertension.

## Patentansprüche

1. Verfahren zur Herstellung von Wasser, das mit natürlicher Orthokieselsäure angereichert ist, wobei das Verfahren mindestens die folgenden aufeinanderfolgenden Schritte umfasst:
a. einen Schritt mit Umkehrosmose eines Rohwassers, das eine Menge an Orthokieselsäure von weniger als 45 mg/l SiO₂ enthält, um ein Retentat zu erhalten;
b. einen Schritt mit Elektrodialyse des in Schritt a) erhaltenen Retentats mit einem Elektrodialysestapel, der alternierende Paare von kationenselektiven Membranen und anionenselektiven Membranen umfasst, wobei die kationenselektiven Membranen und anionenselektiven Membranen aus der Gruppe der monovalenten selektiven Membranen und der Standardgrad-Membranen ausgewählt sind, so dass der Stapel mindestens eine Kationenstandardgradmembran, eine selektive Kationenmonovalentmembran, eine Anionenstandardgradmembran und eine selektive Anionenmonovalentmembran enthält;
c. einen Schritt mit Rückgewinnung eines Endwassers mit einem Orthokieselsäure-Gewichtsanteil von zumindest 150 %, insbesondere zumindest 200 %, weiter insbesondere zumindest 300 % des Orthokieselsäure-Gewichtsanteils des Rohwassers und eines gelösten wertvollen Mineralionen-Gewichtsanteils im Bereich von 70 bis 130 %, insbesondere im Bereich von 80 bis 120 %, weiter insbesondere im Bereich von 90 bis 110 % des gelösten wertvollen Mineralionen-Gewichtsanteils des Rohwassers, wobei die wertvollen Mineralionen unter Calcium, Magnesium, Natrium, Kalium, Bikarbonat, Sulfat und Chloridionen gewählt werden.

2. Verfahren nach Anspruch 1, **dadurch gekennzeichnet, dass** es einen vorbereitenden Schritt a) vor Schritt a) umfasst, der in der Dekontamination des Rohwassers besteht, um ein dekontaminiertes Rohwasser zu erhalten, und dadurch, dass Schritt a) mit dem dekontaminierten Rohwasser durchgeführt wird.

3. Verfahren nach Anspruch 1 oder 2, **dadurch gekennzeichnet, dass** das Rohwasser aus Grundwasser oder Oberflächenwasser, weiter insbesondere Quellwasser, Mineralwasser, natürlichem Mineralwasser oder Trinkwasser ausgewählt wird.

4. Verfahren nach einem der Ansprüche 1 bis 3, **dadurch gekennzeichnet, dass** das Endwasser einen Orthokieselsäuregehalt von zwischen 30 und 120 mg/l SiO₂, insbesondere zwischen 45 und 120 mg/l, weiter insbesondere zwischen 75 und 120 mg/l aufweist.

5. Verfahren nach einem der Ansprüche 1 bis 4, **dadurch gekennzeichnet, dass** das Endwasser einen Trockenrückstand von unter 1500 mg/l, insbesondere unter 1100 mg/l, weiter insbesondere unter 500 mg/l aufweist.

6. Verfahren nach einem der Ansprüche 1 bis 5, **dadurch gekennzeichnet, dass** der Elektrodialysestapel mindestens fünf alternierende Paare von kationenselektiven Membranen und anionenselektiver Membran, insbesondere mindestens 10 alternierende Paare, enthält.

7. Verfahren nach einem der Ansprüche 1 bis 6, **dadurch gekennzeichnet, dass** das Endwasser einen stabilen Orthokieselsäure-Gewichtsanteil bei Lagertemperatur aufweist, und sogar insbesondere bei einer Temperatur unter 10 °C, weiter insbesondere bei etwa 4 °C.

8. Verfahren nach einem der Ansprüche 1 bis 7, **dadurch gekennzeichnet, dass** es einen letzten Schritt d) nach Schritt c) umfasst, der aus der Zugabe des Endwassers zu einem Getränk und/oder einem Sirup und/oder einem Pulver besteht, um ein mit natürlicher bioverfügbarer Orthokieselsäure angereichertes Getränk herzustellen.

9. Verfahren nach einem der Ansprüche 1 bis 8, **dadurch gekennzeichnet, dass** die Rückgewinnungsrate der Umkehrosmose so eingestellt wird, dass die Sättigungsrate der unlöslichen Minerale im Retentat unter 100 %, insbesondere unter 90 %, weiter insbesondere unter 80 % gehalten wird.

10. Verfahren nach einem der Ansprüche 1 bis 9, **dadurch gekennzeichnet, dass** die beiden aufeinanderfolgenden Schritte a) und b) so oft wie nötig an dem nach Schritt b) erhaltenen Wasser wiederholt werden, um ein Endwasser mit dem gewünschten Orthokieselsäure- und gelösten wertvollen Mineralgewichtsanteil zu erhalten, und zwar insbesondere ein- oder zweimal, weiter insbesondere einmal.

11. Verfahren nach Anspruch 10, **dadurch gekennzeichnet, dass** das nach dem ersten Schritt a) der Umkehrosmose erhaltene Retentat nur die Hälfte des gewünschten Orthokieselsäure-Gewichtsanteils des Endwassers enthält.

12. Verfahren nach einem der Ansprüche 1 bis 11, **dadurch gekennzeichnet, dass** die Umkehrosmose-Membrane aus heiß desinfizierbaren Membranen, Meerwasser-Entsalzungsmembranen, Brackwassermembranen, fäuleresistenten Membranen und Niedrigenergiemembranen, insbesondere aus Brackwassermembranen und Meerwasser-Entsalzungsmembranen, ausgewählt werden.

13. Verfahren nach einem der Ansprüche 1 bis 12, **dadurch gekennzeichnet, dass** die Abstoßungsrate von gelösten Mineralionen und Orthokieselsäure im Retentat mindestens höher als 80 %, insbesondere höher als 90 %, weiter insbesondere höher als 95 % ist.

14. Verfahren nach einem der Ansprüche 1 bis 13, **dadurch gekennzeichnet, dass** das Abwasser aus dem Konzentrationsraum des Elektrodialysestapels dem Permeat der Umkehrosmose zur Bildung von Mineraltrinkwasser zugegeben wird.

15. Verfahren nach einem der Ansprüche 1 bis 15, **dadurch gekennzeichnet, dass** der Gehalt an Nitrationen im Endwasser mindestens 80 % des Gewichtsanteils im Rohwasser, insbesondere weniger als 70 %, weiter insbesondere weniger als 50 % beträgt.

16. Stilles Trinkwasser, erhältlich durch das Verfahren nach einem der Ansprüche 1 bis 15, **dadurch gekennzeichnet, dass** es einen gelöstem Natriumgehalt von weniger als 20 mg/l, einen Orthokieselsäuregehalt zwischen 75 und 120 mg/l SiO₂ und einen Trockenrückstand zwischen 400 und 1100 mg/l enthält.

17. Stilles Trinkwasser nach Anspruch 16, **dadurch gekennzeichnet, dass** es weniger als 10 mg/l gelöste Nitrationen enthält.

18. Verwendung des stillen Trinkwassers nach Anspruch 16 oder 17 zur Stärkung und/oder Verschönerung der Haut, des Haars und der Nägel und/oder zur Vorbeugung von Falten und/oder zur Vorbeugung von Hautaustrocknung.

19. Stilles Trinkwasser nach Anspruch 16 oder 17 zur Verwendung als Arzneimittel, insbesondere zur Behandlung und/oder Vorbeugung von Osteoporose, von Aluminiumtoxizität, zur Vorbeugung von neurodegenerativen Erkrankungen, insbesondere der Alzheimer-Krankheit, und/oder zur Vorbeugung von Herz-Kreislauf-Erkrankungen und/oder Arteriosklerose, und/oder zur Vorbeugung von Hauterkrankungen und/oder zur Vorbeugung und/oder Behandlung von Bluthochdruck.

## Revendications

1. Procédé de production d'une eau enrichie en acide orthosilicique naturel, ledit procédé comprenant au moins les étapes successives suivantes :
a. une étape d'osmose inverse d'une eau brute contenant une quantité d'acide orthosilicique inférieure à 45 mg/L de SiO₂ pour obtenir un rétentat ;
b. une étape d'électrodialyse du rétentat obtenu à l'étape a) à l'aide d'une pile d'électrodialyse comprenant des paires alternées de membranes catio-sélectives et de membranes anio-sélectives, lesdites membranes catio-sélectives et membranes anio-sélectives étant choisies dans le groupe des membranes sélectives monovalentes et des membranes de qualité standard de façon que la pile contienne au moins une membrane cationique de qualité standard, une membrane catio-sélective monovalente, une membrane anionique de qualité standard et une membrane anio-sélective monovalente ;
c. une étape de récupération d'une eau finale ayant une teneur en poids d'acide orthosilicique égale à au moins 150 %, en particulier à au moins 200 %, plus particulièrement à au moins 300 % de la teneur en poids d'acide orthosilicique de l'eau brute, et une teneur en poids d'ions minéraux de valeur dissous comprise dans la plage de 70 à 130 %, en particulier dans la plage de 80 à 120 %, plus particulièrement dans la plage de 90 à 110 % de la teneur en poids d'ions minéraux de valeur dissous de l'eau brute, les ions minéraux de valeur étant choisis parmi les ions calcium, magnésium, sodium, potassium, bicarbonate, sulfate et chlorure.

2. Procédé selon la revendication 1, **caractérisé en ce qu'**il comprend une étape préliminaire α) avant l'étape a), consistant en la décontamination de l'eau brute pour obtenir une eau brute décontaminée et **en ce que** l'étape a) est mise en oeuvre sur l'eau brute décontaminée.

3. Procédé selon la revendication 1 ou 2, **caractérisé en ce que** l'eau brute est choisie parmi les eaux souterraines ou les eaux de surface, plus particulièrement une eau de source, une eau minérale, une eau minérale naturelle ou des eaux potables.

4. Procédé selon l'une quelconque des revendications 1 à 3, **caractérisé en ce que** l'eau finale a une teneur en acide orthosilicique comprise entre 30 et 120 mg/L de SiO₂, en particulier entre 45 et 120 mg/L, plus particulièrement entre 75 et 120 mg/L.

5. Procédé selon l'une quelconque des revendications 1 à 4, **caractérisé en ce que** l'eau finale a un résidu sec inférieur à 1500 mg/L, en particulier inférieur à 1100 mg/L, plus particulièrement inférieur à 500 mg/L.

6. Procédé selon l'une quelconque des revendications 1 à 5, **caractérisé en ce que** la pile d'électrodialyse contient au moins cinq paires alternées de membrane catio-sélective et membrane anio-sélective, en particulier au moins 10 paires alternées.

7. Procédé selon l'une quelconque des revendications 1 à 6, **caractérisé en ce que** l'eau finale a une teneur en poids d'acide orthosilicique stable à la température de stockage, même en particulier à une température inférieure à 10 °C, plus particulièrement d'environ 4 °C.

8. Procédé selon l'une quelconque des revendications 1 à 7, **caractérisé en ce qu'**il comprend une étape finale d) après l'étape c), consistant en l'ajout de l'eau finale à une boisson et/ou à un sirop et/ou à une poudre, pour former une boisson enrichie en acide orthosilicique naturel biodisponible.

9. Procédé selon l'une quelconque des revendications 1 à 8, **caractérisé en ce que** le taux de récupération de l'osmose inverse est ajusté pour maintenir le taux de saturation des minéraux insolubles dans le rétentat inférieur à 100 %, en particulier inférieur à 90 %, plus particulièrement inférieur à 80 %.

10. Procédé selon l'une quelconque des revendications 1 à 9, **caractérisé en ce que** les deux étapes successives a) et b) sont répétées sur l'eau obtenue après l'étape b) aussi souvent que nécessaire pour obtenir une eau finale ayant les teneurs en poids d'acide orthosilicique et de minéraux de valeur dissous recherchées, en particulier une ou deux fois, plus particulièrement une fois.

11. Procédé selon la revendication 10, **caractérisé en ce que** le rétentat obtenu après la première étape a) d'osmose inverse ne contient que la moitié de la teneur en poids d'acide orthosilicique recherchée de l'eau finale.

12. Procédé selon l'une quelconque des revendications 1 à 11, **caractérisé en ce que** les membranes d'osmose inverse sont choisies parmi les membranes stérilisables à chaud, les membranes de dessalement d'eau de mer, les membranes pour eau saumâtre, les membranes résistant à l'encrassement et les membranes à basse énergie, en particulier parmi les membranes pour eau saumâtre et les membranes de dessalement d'eau de mer.

13. Procédé selon l'une quelconque des revendications 1 à 12, **caractérisé en ce que** le taux de rejet des ions minéraux dissous et de l'acide orthosilicique dans le rétentat est au moins supérieur à 80 %, en particulier supérieur à 90 %, plus particulièrement supérieur à 95 %.

14. Procédé selon l'une quelconque des revendications 1 à 13, **caractérisé en ce que** l'effluent provenant du compartiment de concentration de la pile d'électrodialyse est ajouté au perméat d'osmose inverse pour former une eau minérale potable.

15. Procédé selon l'une quelconque des revendications 1 à 15, **caractérisé en ce que** la teneur en ion nitrate dans l'eau finale est au moins inférieure à 80 % de sa teneur en poids dans l'eau brute, en particulier inférieure à 70 %, plus particulièrement inférieure à 50 %.

16. Eau plate potable susceptible d'être obtenue par le procédé selon l'une quelconque des revendications 1 à 15, **caractérisée en ce qu'**elle contient une teneur en sodium dissous inférieure à 20 mg/L, une teneur en acide orthosilicique comprise entre 75 et 120 mg/L de SiO₂, et un résidu sec compris entre 400 et 1100 mg/L.

17. Eau plate potable selon la revendication 16, **caractérisée en ce qu'**elle contient moins de 10 mg/L d'ions nitrate dissous.

18. Utilisation de l'eau plate potable selon la revendication 16 ou 17 pour renforcer et/ou embellir la peau, les cheveux et les ongles et/ou pour prévenir les rides et/ou pour prévenir la déshydratation de la peau.

19. Eau plate potable selon la revendication 16 ou 17 pour utilisation à titre de médicament, en particulier pour le traitement et/ou la prévention de l'ostéoporose, de la toxicité de l'aluminium, pour la prévention des maladies neurodégénératives, en particulier de la maladie d'Alzheimer, et/ou pour la prévention des maladies cardiovasculaires et/ou de l'athérosclérose, et/ou pour la prévention des maladies de la peau et/ou pour la prévention et/ou le traitement de l'hypertension.
